(19) Europäisches Patentamt
European Patent Office
Office européen des brevets

(11) **EP 4 442 736 A1**

(12) **EUROPEAN PATENT APPLICATION**
published in accordance with Art. 153(4) EPC

(43) Date of publication:
**09.10.2024 Bulletin 2024/41**

(21) Application number: **22922138.7**

(22) Date of filing: **15.12.2022**

(51) International Patent Classification (IPC):
*C08J 3/12* (2006.01)     *A61K 8/00* (2006.01)
*A61K 8/73* (2006.01)     *A61K 8/81* (2006.01)
*A61K 8/85* (2006.01)     *A61K 8/86* (2006.01)
*A61K 8/87* (2006.01)     *A61K 8/88* (2006.01)
*C08J 3/16* (2006.01)     *C09D 7/65* (2018.01)
*C09D 201/00* (2006.01)

(52) Cooperative Patent Classification (CPC):
**A61K 8/00; A61K 8/73; A61K 8/81; A61K 8/85;
A61K 8/86; A61K 8/87; A61K 8/88; C08J 3/12;
C08J 3/16; C09D 7/65; C09D 201/00**

(86) International application number:
**PCT/JP2022/046119**

(87) International publication number:
**WO 2023/139987 (27.07.2023 Gazette 2023/30)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.01.2022   JP 2022005385
28.09.2022   JP 2022154346**

(71) Applicant: **Matsumoto Yushi-Seiyaku Co., Ltd.
Yao-shi, Osaka 581-0075 (JP)**

(72) Inventor: **TOKUMURA, Sachiko
Yao-shi, Osaka 581-0075 (JP)**

(74) Representative: **Prüfer & Partner mbB
Patentanwälte · Rechtsanwälte
Sohnckestraße 12
81479 München (DE)**

(54) **PARTICLES AND USE THEREOF**

(57)   The purpose of the present invention is to provide particles having excellent slipperiness and a soft texture, and uses thereof. (Particles of first embodiment) Particles having an average particle diameter of 0.5-100 $\mu$m, a compressive aggregate modulus of 0-25% and a compressive recovery modulus of 60-100%. Preferably, the particles have a sphericity of 0.6-1.0. Preferably, the particles contain an organic polymer. Preferably, the organic polymer contains a thermoplastic resin. (Particles of second embodiment) Particles containing a thermoplastic resin and having an average particle diameter of 0.5-100 $\mu$m and a compressive aggregate modulus of 0-25%.

FIG. 1

**Description**

TECHNICAL FIELD

The present invention relates to particles and a use thereof.

BACKGROUND ART

Particles are often used in cosmetics, paints, optical applications, resins, building materials, and the like. Examples of a function required for the particles include a light diffusion property, a hiding property, a coating property, and texture impartion, and examples of a property required of the particles include a refractive index, dispersibility, slipperiness, and flexibility. For example, in cosmetics, paints, or the like, particles having a soft texture in terms of imparting a texture and the like are preferred. In recent years, as concerns about the environment have increased, there has been a demand for particles that have less environmental impact, and biodegradable particles are attracting particular attention.

Patent Literature 1 describes a method for producing polylactic acid resin microparticles made of polylactic acid derived from a non-petroleum raw material and polylactic acid resin microparticles as particles for reducing an environmental impact. Patent Literature 2 describes porous resin microparticles made of a polyester thermoplastic resin having biodegradability.

CITATION LIST

PATENT LITERATURE

Patent Literature 1: WO2012/105140
Patent Literature 2: WO2017/056908

SUMMARY OF INVENTION

TECHNICAL PROBLEM

However, these particles are not satisfactory in terms of a coating property, slipperiness, and a texture.

Therefore, an object of the present invention is to provide particles having an excellent slipperiness and a soft texture.

SOLUTION TO PROBLEM

As a result of intensive studies to achieve the above object, the inventor of the present application has found that particles having an excellent slipperiness and a soft texture can be obtained by causing the particles to exhibit specific physical properties.

That is, particles according to a first aspect of the present invention have an average particle diameter of 0.5 $\mu$m to 100 $\mu$m, a compressive aggregate modulus of 0% to 25%, and a compressive recovery modulus of 60% to 100%.

The particles according to the first aspect of the present invention preferably satisfy at least one of the following 1) to 6).

1) The particles contain an organic polymer.
2) The organic polymer includes a thermoplastic resin.
3) The thermoplastic resin includes at least one selected from a polyvinyl resin, a polyacrylic resin, a polystyrene resin, a polyolefin resin, a polyester resin, a polyether resin, a polyamide resin, a thermoplastic polyurethane resin, and a cellulose resin.
4) The particles have a sphericity of 0.6 to 1.0.
5) A value (D90/D50) obtained by dividing a particle diameter (D90) at a cumulative frequency of 90% by volume-based measurement by the average particle diameter (D50) is 1.0 to 3.5.
6) The particles have biodegradability.

Particles according to a second aspect of the present invention include a thermoplastic resin, and have an average particle diameter of 0.5 $\mu$m to 100 $\mu$m and a compressive aggregate modulus of 0% to 25%.

The particles according to the second aspect of the present invention preferably satisfy at least one of the following 7) to 10).

7) The thermoplastic resin includes at least one selected from a polyvinyl resin, a polyacrylic resin, a polystyrene resin, a polyolefin resin, a polyester resin, a polyether resin, a polyamide resin, a thermoplastic polyurethane resin, and a cellulose resin.

8) The particles have a sphericity of 0.6 to 1.0.

9) A value (D90/D50) obtained by dividing a particle diameter (D90) at a cumulative frequency of 90% by volume-based measurement by the average particle diameter (D50) is 1.0 to 3.5.

10) The particles have biodegradability.

A cosmetic according to the present invention contains the particles.

A coating composition according to the present invention contains the particles.

ADVANTAGEOUS EFFECTS OF INVENTION

The particles according to the present invention have an excellent slipperiness and a soft texture.

Since the cosmetic according to the present invention contains the particles, the cosmetic has an excellent slipperiness and a soft texture.

Since the coating composition according to the present invention contains the particles, the coating composition has an excellent slipperiness and a soft texture.

BRIEF DESCRIPTION OF DRAWINGS

[FIG. 1] FIG. 1 is a photograph showing a state of particles of Example 1 after a coating test.

[FIG. 2] FIG. 2 is a photograph showing a state of particles of Comparative Example 1 after a coating test.

DESCRIPTION OF EMBODIMENTS

The particles according to the first aspect of the present invention have an average particle diameter (D50) of 0.5 $\mu$m to 100 $\mu$m, a compressive aggregate modulus of 0% to 25%, and a compressive recovery modulus of 60% to 100%.

The average particle diameter (D50) of the particles according to the first aspect of the present invention is 0.5 $\mu$m to 100 $\mu$m. In the case where the average particle diameter is less than 0.5 $\mu$m, slipperiness and smoothness are poor. On the other hand, in the case where the average particle diameter (D50) is more than 100 $\mu$m, roughness is felt and a soft feeling is poor. An upper limit of the particle diameter is preferably 50 $\mu$m, more preferably 40 $\mu$m, still more preferably 30 $\mu$m, and particularly preferably 20 $\mu$m. On the other hand, a lower limit of the particle diameter is preferably 1 $\mu$m, more preferably 1.5 $\mu$m, still more preferably 2.5 $\mu$m, and particularly preferably 3 $\mu$m. Furthermore, for example, the average particle diameter is preferably 1 $\mu$m to 50 $\mu$m, and more preferably 1.5 $\mu$m to 30 $\mu$m. The average particle diameter (D50) is a value at a cumulative frequency of 50% by the volume-based measurement. The average particle diameter (D50) of the particles is determined by the method described in Examples.

The compressive aggregate modulus of the particles according to the first aspect of the present invention is 0% to 25%. In the case where the compressive aggregate modulus is more than 25%, an aggregation force among the particles is strong, and the slipperiness and the coating property are poor. The compressive aggregate modulus indicates an aggregation property among the particles when a pressure is applied to a powder layer of the particles. It is considered that particles having a high compressive aggregate modulus tend to aggregate when the pressure is applied, and the particles tend to aggregate and form lumps due to the pressure during application, and thus the particles have a poor slipperiness and are difficult to apply uniformly, resulting in the poor coating property. An upper limit of the compressive aggregate modulus is preferably 20%, more preferably 17%, still more preferably 15%, and particularly preferably 12%. Further, for example, 0% to 20% is more preferable, and 0% to 17% is still more preferable.

The compressive recovery modulus of the particles according to the first aspect of the present invention is 60% to 100%. In the case where the compressive recovery modulus is less than 60%, the soft texture is poor because hardness is felt. The compressive recovery modulus indicates a degree of displacement of the powder layer of the particles due to pressurization and depressurization. It is considered that particles having a high compressive recovery modulus have a soft texture since the particles and the powder layer have elasticity. A lower limit of the compressive recovery modulus is preferably 65%, more preferably 70%, still more preferably 75%, and particularly preferably 80%.

Furthermore, for example, 65% to 100% is more preferable, and 70% to 100% is still more preferable.

A method for measuring the compressive aggregate modulus and the compressive recovery modulus according to the present invention is in accordance with the method described in Examples.

The particles according to the first aspect of the present invention are not particularly limited, but in the case where the value (D90/D50) obtained by dividing the particle diameter (D90) at a cumulative frequency of 90% by the volume-based measurement by the average particle diameter (D50) is 1.0 to 3.5, the compressive aggregate modulus and the compressive recovery modulus easily satisfy the above range, and the slipperiness is more excellent, which is preferable. An upper limit of the D90/D50 is more preferably 3.0, still more preferably 2.8, and particularly preferably 2.5. On the other hand, a lower limit of the D90/D50 is more preferably 1.1, still more preferably 1.3, and particularly preferably 1.5. Further, for example, 1.1 to 3.0 is more preferable, 1.3 to 2.8 is still more preferable, and 1.3 to 2.5 is particularly preferable.

Further, in the case where a value (D10/D50) obtained by dividing a particle diameter (D10) at a cumulative frequency of 10% by the volume-based measurement by the average particle diameter (D50) is 0.1 to 1.0, the compressive aggregate modulus and the compressive recovery modulus easily satisfy the above range, and the slipperiness is more excellent, which is preferable. An upper limit of the D10/D50 is more preferably 0.9, still more preferably 0.8, and particularly preferably 0.7. On the other hand, a lower limit of the D10/D50 is more preferably 0.2, still more preferably 0.25, and particularly preferably 0.3. Further, for example, 0.1 to 0.9 is more preferable, 0.2 to 0.8 is still more preferable, and 0.3 to 0.7 is particularly preferable.

A method for measuring the D10 and D90 is in accordance with the method described in Examples.

A coefficient of variation CV of a particle size distribution of the particles according to the first aspect of the present invention is not particularly limited, but is preferably 2% to 70% in terms of the excellent slipperiness. An upper limit of the coefficient of variation CV is preferably 65%, more preferably 60%, still more preferably 55%, and particularly preferably 50%. A lower limit of the coefficient of variation CV is preferably 3%, more preferably 5%, and particularly preferably 7%. Further, for example, 3% to 65% is more preferable, and 5% to 60% is still more preferable. The coefficient of variation CV is calculated by the following Equations (1) and (2).

$$CV = (s / <x>) \times 100 \ (\%) \qquad \cdots (1)$$

$$s = \left\{ \sum_{i=1}^{n} (x_i - <x>)^2 / (n-1) \right\}^{1/2} \qquad \cdots (2)$$

(In the equations, s is a standard deviation of a particle diameter, <x> is an average particle diameter, $x_i$ is an i-th particle diameter, and n is the number of particles.)

A sphericity of the particles according to the first aspect of the present invention is not particularly limited, but is preferably 0.6 to 1.0 in terms of the excellent slipperiness. A lower limit of the sphericity is preferably in the order of (1) 0.65, (2) 0.70, (3) 0.75, (4) 0.80, (5) 0.85, and (6) 0.90 (the larger the number in the parentheses, the better). Furthermore, for example, 0.65 to 1.0 is more preferable, and 0.70 to 1.0 is still more preferable. The sphericity of the particles according to the present invention is determined by the method described in Examples.

The particles according to the first aspect of the present invention are not particularly limited, but it is preferable that the particles contain an organic polymer in terms of an excellent soft texture.

A weight ratio of the organic polymer in the particles is not particularly limited, but is preferably 1% by weight to 100% by weight. An upper limit of the weight ratio is more preferably 99.9% by weight, still more preferably 99.5% by weight, and particularly preferably 99.0% by weight. On the other hand, a lower limit of the weight ratio is preferably in the order of (1) 5% by weight, (2) 10% by weight, (3) 20% by weight, (4) 30% by weight, (5) 40% by weight, (6) 50% by weight, and (7) 60% by weight (the larger the number in the parentheses, the better). Furthermore, for example, 10% by weight to 100% by weight is more preferable, and 30% by weight to 100% by weight is still more preferable.

The organic polymer is not particularly limited, but it is preferable that a weight average molecular weight is $5 \times 10^3$ to $1 \times 10^9$ in terms of the excellent slipperiness. A lower limit of the average molecular weight is preferably in the order of (1) $1 \times 10^4$, (2) $2 \times 10^4$, (3) $3 \times 10^4$, (4) $5 \times 10^4$, (5) $1 \times 10^5$, (6) $2 \times 10^5$, and (7) $3 \times 10^5$. On the other hand, an upper limit of the average molecular weight is preferably in the order of (1) $5 \times 10^8$, (2) $3 \times 10^8$, (3) $1 \times 10^8$, (4) $5 \times 10^7$, (5) $3 \times 10^7$, and (6) $1 \times 10^7$ (the larger the number in the parentheses, the better). Further, for example, $2 \times 10^4$ to $5 \times 10^8$ is more preferable, and $3 \times 10^4$ to $5 \times 10^8$ is still more preferable.

The organic polymer preferably includes at least one selected from, for example, a thermoplastic resin, a thermosetting resin, and a cellulose. Among them, it is preferable to include a thermoplastic resin since a soft texture can be provided, and it is preferable to include a cellulose since biodegradability can be provided.

In the case where the organic polymer includes a thermoplastic resin, a weight ratio of the thermoplastic resin to the organic polymer is not particularly limited, but is preferably 1% by weight to 100% by weight. An upper limit of the weight ratio is more preferably 99.9% by weight, still more preferably 99.5% by weight, and particularly preferably 99.0% by weight. On the other hand, a lower limit of the weight ratio is preferably in the order of (1) 5% by weight, (2) 10% by weight, (3) 20% by weight, (4) 25% by weight, (5) 30% by weight, (6) 40% by weight, and (7) 50% by weight (the larger the number in the parentheses, the better). Furthermore, for example, 10% by weight to 100% by weight is more preferable, and 30% by weight to 100% by weight is still more preferable.

In the case where the organic polymer includes the cellulose, a weight ratio of the cellulose to the organic polymer is not particularly limited, but is preferably 1% by weight to 100% by weight. An upper limit of the weight ratio is more preferably 99% by weight, still more preferably 95% by weight, and particularly preferably 90% by weight. On the other hand, a lower limit of the weight ratio is preferably in the order of (1) 3% by weight, (2) 5% by weight, (3) 10% by weight, (4) 20% by weight, (5) 30% by weight, (6) 40% by weight, and (7) 50% by weight (the larger the number in the parentheses, the better). Furthermore, for example, 3% by weight to 99% by weight is more preferable, and 5% by weight to 99% by weight is still more preferable.

The particles according to the second aspect of the present invention are particles including a thermoplastic resin and having an average particle diameter of 0.5 $\mu$m to 100 $\mu$m and a compressive aggregate modulus of 0% to 25%.

The particles according to the second aspect of the present invention include the thermoplastic resin.

A weight ratio of the thermoplastic resin in the particles according to the second aspect of the present invention is not particularly limited, but is preferably 1% by weight to 100% by weight. The thermoplastic resin is a resin having a property of being plasticized by heat, and has a property that molecular motion is more likely to occur due to an influence of an external temperature. Therefore, it is considered that a soft texture can be obtained when a user touches the particles with a hand thereof. An upper limit of the weight ratio is more preferably 99.9% by weight, still more preferably 99.5% by weight, and particularly preferably 99.0% by weight. On the other hand, a lower limit of the weight ratio is preferably in the order of (1) 5% by weight, (2) 10% by weight, (3) 20% by weight, (4) 25% by weight, (5) 30% by weight, (6) 40% by weight, and (7) 50% by weight (the larger the number in the parentheses, the better). Furthermore, for example, 10% by weight to 100% by weight is more preferable, and 30% by weight to 100% by weight is still more preferable.

The thermoplastic resin is not particularly limited, but it is preferable that a weight average molecular weight is $5 \times 10^3$ to $1 \times 10^9$ in terms of the excellent slipperiness. A lower limit of the average molecular weight is preferably in the order of (1) $1 \times 10^4$, (2) $2 \times 10^4$, (3) $3 \times 10^4$, (4) $5 \times 10^4$, (5) $1 \times 10^5$, (6) $2 \times 10^5$, and (7) $3 \times 10^5$. On the other hand, an upper limit of the average molecular weight is preferably in the order of (1) $5 \times 10^8$, (2) $3 \times 10^8$, (3) $1 \times 10^8$, (4) $5 \times 10^7$, (5) $3 \times 10^7$, and (6) $1 \times 10^7$ (the larger the number in the parentheses, the better). Further, for example, $2 \times 10^4$ to $5 \times 10^8$ is more preferable, and $3 \times 10^4$ to $5 \times 10^8$ is still more preferable.

The average particle diameter (D50) of the particles according to the second aspect of the present invention is 0.5 $\mu$m to 100 $\mu$m. In the case where the average particle diameter (D50) is less than 0.5 $\mu$m, slipperiness and smoothness are poor. On the other hand, in the case where the average particle diameter (D50) is more than 100 $\mu$m, roughness is felt and a soft feeling is poor. An upper limit of the average particle diameter is preferably 50 $\mu$m, more preferably 40 $\mu$m, still more preferably 30 $\mu$m, and particularly preferably 20 $\mu$m. On the other hand, a lower limit of the average particle diameter is preferably 1 $\mu$m, more preferably 1.5 $\mu$m, still more preferably 2.5 $\mu$m, and particularly preferably 3 $\mu$m. Furthermore, for example, the average particle diameter is preferably 1 $\mu$m to 50 $\mu$m, and more preferably 1.5 $\mu$m to 30 $\mu$m.

The average particle diameter (D50) is a value at a cumulative frequency of 50% by the volume-based measurement.

The average particle diameter of the particles is determined by the method described in Examples.

The compressive aggregate modulus of the particles according to the second aspect of the present invention is 0% to 25%. In the case where the compressive aggregate modulus is more than 25%, an aggregation force among the particles is strong, and the slipperiness and the coating property are poor. The compressive aggregate modulus indicates an aggregation property among the particles when a pressure is applied to a powder layer of the particles. It is considered that particles having a high compressive aggregate modulus tend to aggregate when the pressure is applied, and the particles tend to aggregate and form lumps due to the pressure during application, and thus the particles have a poor slipperiness and are difficult to apply uniformly, resulting in the poor coating property. An upper limit of the compressive aggregate modulus is preferably 20%, more preferably 17%, still more preferably 15%, and particularly preferably 12%. Further, for example, 0% to 20% is more preferable, and 0% to 17% is still more preferable.

The compressive recovery modulus of the particles according to the second aspect of the present invention is preferably 60% to 100% in terms of an excellent soft texture. A lower limit of the compressive recovery modulus is preferably 65%, more preferably 70%, still more preferably 75%, and particularly preferably 80%. Furthermore, for example, 65% to 100% is more preferable, and 70% to 100% is still more preferable. A method for measuring the compressive aggregate modulus and the compressive recovery modulus according to the present invention is in accordance with the method described in Examples.

The particles according to the second aspect of the present invention are not particularly limited, but in the case where the value (D90/D50) obtained by dividing the particle diameter (D90) at a cumulative frequency of 90% by the volume-based measurement by the average particle diameter (D50) is 1.0 to 3.5, the compressive aggregate modulus and the compressive recovery modulus easily satisfy the above range, and the slipperiness is more excellent, which is preferable. An upper limit of the D90/D50 is more preferably 3.0, still more preferably 2.8, and particularly preferably 2.5. On the other hand, a lower limit of the D90/D50 is more preferably 1.1, still more preferably 1.3, and particularly preferably 1.5. Further, for example, 1.1 to 3.0 is more preferable, 1.3 to 2.8 is still more preferable, and 1.3 to 2.5 is particularly preferable.

Further, in the case where a value (D10/D50) obtained by dividing a particle diameter (D10) at a cumulative frequency of 10% by the volume-based measurement by the average particle diameter (D50) is 0.1 to 1.0, the compressive aggregate modulus and the compressive recovery modulus easily satisfy the above range, and the slipperiness is more excellent, which is preferable. An upper limit of the D10/D50 is more preferably 0.9, still more preferably 0.8, and particularly preferably 0.7. On the other hand, a lower limit of the D10/D50 is more preferably 0.2, still more preferably 0.25, and particularly preferably 0.3. Further, for example, 0.1 to 0.9 is more preferable, 0.2 to 0.8 is still more preferable, and 0.3 to 0.7 is particularly preferable.

The coefficient of variation CV of a particle size distribution of the particles according to the second aspect of the present invention is not particularly limited, but is preferably 2% to 70% in terms of the excellent slipperiness. An upper limit of the coefficient of variation CV is preferably 65% or less, more preferably 60% or less, still more preferably 55% or less, and particularly preferably 50% or less. A lower limit of the coefficient of variation CV is preferably 3%, more preferably 5%, and particularly preferably 7%. Further, for example, 3% to 65% is more preferable, and 5% to 60% is still more preferable.

The coefficient of variation CV is calculated by the following Equations (1) and (2).

$$CV = (s/<x>) \times 100 \quad (\%) \qquad \cdots (1)$$

$$s = \left\{ \sum_{i=1}^{n} (x_i - <x>)^2 / (n-1) \right\}^{1/2} \qquad \cdots (2)$$

(In the equations, s is a standard deviation of a particle diameter, $<x>$ is an average particle diameter, $x_i$ is an i-th particle diameter, and n is the number of particles.)

The sphericity of the particles according to the second aspect of the present invention is not particularly limited, but is preferably 0.6 to 1.0 in terms of the excellent slipperiness. A lower limit of the sphericity is preferably in the order of (1) 0.65, (2) 0.70, (3) 0.75, (4) 0.80, (5) 0.85, and (6) 0.90 (the larger the number in the parentheses, the better). The sphericity of the particles according to the present invention is determined by the method described in Examples. Furthermore, for example, 0.65 to 1.0 is more preferable, and 0.70 to 1.0 is still more preferable.

The particles according to the second aspect of the present invention may further include an organic polymer such as a thermosetting resin or a cellulose in addition to the thermoplastic resin.

Examples of the thermoplastic resin include a polyvinyl resin, a polyacrylic resin, a polystyrene resin, a polyolefin resin, a polyester resin, a polyether resin, a polyamide resin, a thermoplastic polyurethane resin, and a cellulose resin. These thermoplastic resins may be used alone or in combination of two or more thereof. Among these thermoplastic resins, a polyester resin and/or a cellulose resin are preferable and a polyester resin is more preferable in terms of biodegradability. By using these resins, it becomes easier to obtain particles having a low compressive aggregate modulus.

Examples of the polyvinyl resin include polyvinyl chloride, polyvinyl acetate, polyvinyl alcohol, and the like. These polyvinyl resins may be used alone or in combination of two or more thereof.

Examples of the polyacrylic resin include polymethylmethacrylate, poly(meth)acrylic acid ester, a poly(meth)acrylic acid ester/acrylic acid copolymer, and the like. These polyacrylic resins may be used alone or in combination of two or more thereof.

Examples of the polystyrene resin include polystyrene, a poly(meth)acrylic acid ester/styrene copolymer, and a polystyrene elastomer. These polystyrene resins may be used alone or in combination of two or more thereof.

Examples of the polyolefin resin include polyethylene, polypropylene, an ethylene/vinyl acetate copolymer, an ethylene/(meth)acrylic acid copolymer, an ethylene/(meth)acrylic acid ester copolymer, a low molecular weight polyolefin, and a polyolefin elastomer. These polyolefin resins may be used alone or in combination of two or more thereof.

Examples of the polyester resin include a polycondensate of polyhydric alcohols and polycarboxylic acids, and a polycondensate of polyhydric alcohols, polycarboxylic acids, and hydroxy carboxylic acids, a polycondensate of hydroxy carboxylic acids, and a polycondensate of derivatives thereof.

Examples of the polyester resin include polylactic acid, polypropiolactone, polycaprolactone, polycaprolactone butylene succinate, polybutylene adipate caprolactone, polybutylene succinate hydroxycaproate, polybutylene succinate, polybutylene succinate adipate, polybutylene succinate carbonate, polybutylene succinate terephthalate, polybutylene succinate adipate terephthalate, polybutylene succinate lactate, polyethylene terephthalate, polytrimethylene terephthalate, a polyethylene terephthalate copolymer, polybutylene adipate terephthalate, polytetramethylene adipate terephthalate, polybutylene terephthalate, polyethylene naphthalate, polybutylene naphthalate, polybutylene adipate, polybutylene adipate terephthalate, polyethylene succinate, polyethylene adipate, polyethylene adipate terephthalate, polytetramethylene succinate, polypropylene succinate, polyhydroxyalkanoate, polyhydroxybutyrate, poly(3-hydroxybutyrate-co-3-hydroxy hexanoate), polyhydroxybutyrate valerate, poly(3-hydroxybutyrate-co-3-hydroxyvalerate), poly(3-hydroxybutyrate-co-3-hydroxypropionate), poly(3-hydroxybutyrate-co-4-hydroxybutyrate), poly(3-hydroxybutyrate-co-3-hydroxy acyl), polyhydroxyacyl, polyglycolic acid, a lactic acid-glycolic acid copolymer, and a polyester elastomer. These polyester resins may be used alone or in combination of two or more thereof.

Examples of the polyester resin include an aliphatic polyester resin, an aromatic polyester resin, and an aliphatic-aromatic polyester resin, and in terms of excellent biodegradability, an aliphatic polyester resin and an aliphatic-aromatic polyester resin are preferred, and an aliphatic polyester resin are particularly preferred.

The aliphatic polyester resin is not particularly limited as long as the polyhydric alcohols, the polycarboxylic acids, and the hydroxy carboxylic acids, which are constituent components of the polyester resin, are aliphatic polyhydric alcohols, aliphatic polycarboxylic acids, and aliphatic hydroxy carboxylic acids, respectively.

Examples of the aliphatic polyhydric alcohol include ethylene glycol, 1,3-propanediol, 1,4-butanediol, diethylene glycol, 1,5-pentanediol, 1,6-hexanediol, propylene glycol, dipropylene glycol, triethylene glycol, tetraethylene glycol, 1,2-propanediol, 1,3-butanediol, 2,3-butanediol, neopentylglycol (2,2-dimethylpropane-1,3-diol), 1,2-hexanediol, 2,5-hexanediol, 2-methyl-2,4-pentanediol, 3-methyl-1,3-pentanediol, 2-ethyl-1,3-hexanediol, 2,2-bis(4-hydroxycyclohexyl)propane, 1,4-cyclohexanediol, 1,4-cyclohexane dimethanol, trimethylol propan, glycerin, and pentaerythritol. These aliphatic polyhydric alcohols may be used alone or in combination of two or more thereof.

Examples of the aliphatic polycarboxylic acid include succinic acid, adipic acid, suberic acid, sebacic acid, azelaic acid, octylsuccinic acid, fumaric acid, maleic acid, itaconic acid, decamethylene dicarboxylic acid, and anhydrides thereof. These aliphatic polycarboxylic acids may be used alone or in combination of two or more thereof.

Examples of the aliphatic hydroxy carboxylic acid include lactic acid, glycolic acid, hydroxybutyric acid, hydroxycaproic acid, hydroxydimethyl butyric acid, and hydroxymethyl butyric acid. These aliphatic hydroxy carboxylic acids may be used alone or in combination of two or more thereof.

Examples of the aliphatic polyester resin include polycaprolactone butylene succinate, polybutylene succinate, polybutylene succinate adipate, polybutylene succinate carbonate, polybutylene adipate, polybutylene succinate lactate, polyethylene succinate, polyethylene adipate, polytetramethylene succinate, polyhydroxyalkanoate, polyhydroxybutyrate, poly(3-hydroxybutyrate-co-3-hydroxy hexanoate), polyhydroxybutyrate valerate, poly(3-hydroxybutyrate-co-3-hydroxyvalerate), poly(3-hydroxybutyrate-co-3-hydroxypropionate), poly(3-hydroxybutyrate-co-4-hydroxybutyrate), poly(3-hydroxybutyrate-co-3-hydroxy acyl), polyhydroxyacyl, and polylactic acid.

The aliphatic-aromatic polyester resin is not particularly limited as long as the polyhydric alcohols, the polycarboxylic acids, and the hydroxy carboxylic acids, which are constituent components of the polyester resin, include the aliphatic polyhydric alcohols, the aliphatic polycarboxylic acids, and the aliphatic hydroxy carboxylic acids, respectively, and further the aliphatic-aromatic polyester resin include aromatic polycarboxylic acids or derivatives thereof.

Examples of the aromatic polycarboxylic acid include o-phthalic acid, terephthalic acid, isophthalic acid, cyclohexanedicarboxylic acid, naphthalenedicarboxylic acid, diphenyldicarboxylic acid, trimellitic acid, and pyromellitic acid. In terms of the biodegradability, a proportion of a constituent component derived from the aromatic polycarboxylic acid in constituent components of the aliphatic-aromatic polyester resin is preferably 40 unit mol% or less. These aromatic polycarboxylic acids may be used alone or in combination of two or more thereof.

Examples of the aliphatic-aromatic polyester resin include polybutylene succinate terephthalate, polybutylene adipate terephthalate, polytetramethylene adipate terephthalate, and polybutylene succinate adipate terephthalate. These aliphatic-aromatic polyester resins may be used alone or in combination of two or more thereof.

Examples of the polyether resin include polyphenylene ether, polysulfone, polyethersulfone, polyacetal, polyether ketone, and polyether ether ketone. These polyether resins may be used alone or in combination of two or more thereof.

Examples of the polyamide resin include nylon 1, nylon 3, nylon 4, polycaproamide (nylon 6), poly-co-aminoheptanoic acid (nylon 7), poly-9-aminononanoic acid (nylon 9), polyundecanamide (nylon 11), polylauryllactam (nylon 12), polyethylene diamine adipamide (nylon 2, 6), polytetramethylene adipamide (nylon 4, 6), polyhexamethylene diadipamide (nylon 6, 6), polyhexamethylene sebacamide (nylon 6, 10), polyhexamethylene dodecamide (nylon 6, 12), polyoctamethylene adipamide (nylon 8, 6), polydecamethylene adipamide (nylon 10, 6), polydecamethylene sebacamide (nylon 10, 10), polydodecamethylene dodecamide (nylon 12, 12), and metaxylene diamine-6 nylon (MXD6). These polyamide resins may be used alone or in combination of two or more thereof.

Examples of the thermoplastic polyurethane resin include a polyester polyurethane resin, a polyether polyurethane resin, a polycarbonate polyurethane resin. These thermoplastic polyurethane resins may be used alone or in combination of two or more thereof.

Examples of the cellulose resin include cellulose acetate, ethyl cellulose, a cellulose ether derivative, cellulose acetate propionate, and cellulose acetate butyrate. These cellulose resins may be used alone or in combination of two or more thereof.

A melting point or a softening point of the thermoplastic resin is not particularly limited, but it is preferable that either the melting point or the softening point be 40°C to 200°C, since a texture is excellent when the particles are applied. A lower limit of the melting point or the softening point of the thermoplastic resin is more preferably 50°C, still more preferably 60°C, and most preferably 70°C. An upper limit of the melting point or the softening point of the thermoplastic resin is more preferably 190°C, still more preferably 180°C, and most preferably 165°C. Further, for example, 60°C to 200°C is more preferable, and 60°C to 180°C is still more preferable.

Examples of the thermosetting resin include a polyurethane resin, a silicone resin, a phenolic resin, an unsaturated polyester resin, an epoxy resin, a melamine resin, and rubber. The thermosetting resins may be used alone or in combination of two or more thereof.

The organic polymer may include an organic polymer other than the thermoplastic resin, the thermosetting resin, and the cellulose (hereinafter sometimes referred to as another organic polymer).

Examples of the other organic polymer include paraffins, silicone oils, polyalkylene oxides, and water-soluble polymers, and specific examples thereof include paraffins such as liquid paraffin; silicone oils such as dimethyl silicone; polyalkylene oxides such as a polyethylene oxide and a polypropylene oxide; and water-soluble polymers such as sodium polyacrylate, polyvinylpyrrolidone, polyvinyl alcohol, dextrin, sodium alginate, potassium alginate, gum arabic, tamarind gum, pectin, pullulan, casein, xanthan gum, carrageenan, gum tragacanth, gelatin, hydroxyethyl cellulose, hydroxypropyl cellulose, methyl cellulose, carboxymethyl cellulose, and carboxyethyl cellulose.

The other organic polymer preferably includes a water-soluble polymer in terms of reducing the compressive aggregate modulus.

The water-soluble polymer preferably includes at least one selected from polyvinylpyrrolidone, polyvinyl alcohol, hydroxyethyl cellulose, hydroxypropyl cellulose, methyl cellulose, and carboxymethyl cellulose in terms of reducing the compressive aggregate modulus, and the water-soluble polymer more preferably includes polyvinyl alcohol. These other organic polymers may be used alone or in combination of two or more thereof.

In the case where the thermoplastic resin contains the polyester resin, a weight ratio of the polyester resin to the thermoplastic resin is not particularly limited, but is preferably 1% by weight to 100% by weight. An upper limit of the weight ratio is more preferably 99.9% by weight, still more preferably 99.5% by weight, and particularly preferably 99.0% by weight. On the other hand, a lower limit of the weight ratio is preferably in the order of (1) 5% by weight, (2) 10% by weight, (3) 20% by weight, (4) 30% by weight, (5) 40% by weight, (6) 50% by weight, and (7) 60% by weight (the larger the number in the parentheses, the better). Furthermore, for example, 10% by weight to 100% by weight is more preferable, and 30% by weight to 100% by weight is still more preferable.

In the case where the polyester resin contains the aliphatic polyester resin, a weight ratio of the aliphatic polyester resin to the polyester resin is not particularly limited, but is preferably 1% by weight to 100% by weight. An upper limit of the weight ratio is more preferably 99.9% by weight, still more preferably 99.5% by weight, and particularly preferably 99.0% by weight. On the other hand, a lower limit of the weight ratio is preferably in the order of (1) 3% by weight, (2) 5% by weight, (3) 10% by weight, (4) 20% by weight, (5) 30% by weight, (6) 40% by weight, and (7) 50% by weight (the larger the number in the parentheses, the better). Furthermore, for example, 5% by weight to 100% by weight is more preferable, and 20% by weight to 100% by weight is still more preferable.

It is particularly preferable that the weight ratio of the aliphatic polyester resin in the polyester resin be 50% by weight or more, since a soft feel can be obtained and biodegradability can be provided.

The particles according to the present invention may contain at least one selected from surfactants, organic materials other than the organic polymer, and inorganic materials.

Examples of the surfactant include anionic surfactants, cationic surfactants, nonionic surfactants, amphoteric surfactants, and silicone surfactants, and specific examples thereof include anionic surfactants such as alkyl sulfates, alkyl ether carboxylates, alkyl ether sulfates, alkyl phosphates, polyoxyalkylene alkyl ether acetates, alkyl sulfonates, alkyl benzene sulfonates, polyoxyalkylene alkyl ether sulfates, polyoxyalkylene alkyl ether phosphates, higher fatty acid amide sulfonates, fatty acid alkali metal salts (for example, potassium laurate, sodium myristate, and sodium stearate), alkyl sulfosuccinates, N-acylamino acid salts; cationic surfactants such as quaternary ammonium salts, and alkylamine salts; nonionic surfactants such as polyoxyalkylene oxide-added alkyl ether, polyoxyalkylene styrenated phenyl ether, an ester compound of a polyhydric alcohol and a monohydric fatty acid, polyoxyalkylene alkylphenyl ether, polyoxyalkylene fatty acid ester, polyoxyalkylene sorbitan fatty acid ester, glycerin fatty acid ester, polyoxyalkylene castor oil, polyoxyalkylene hydrogenated castor oil, higher fatty acid PEG glyceryl, sorbitan higher fatty acid ester, polyoxyalkylene sorbitol-fatty acid ester, polyglycerin fatty acid ester, alkyl glycerin ether, polyoxyalkylene cholesteryl ether, alkylpolyglucoside, sucrose fatty acid ester, and polysorbates; amphoteric surfactants such as amino acid surfactants, betaine surfactants, hydrogenated lecithin, and lecithin; and silicone surfactants such as modified silicone.

The surfactant preferably contains at least one selected from the anionic surfactants and the nonionic surfactants in terms of reducing the compressive aggregate modulus, and more preferably contains the nonionic surfactants.

The anionic surfactant preferably contains at least one selected from sulfates and sulfonates, and sulfonates are more preferable.

The nonionic surfactant preferably contains an ester compound of a polyhydric alcohol and a monohydric fatty acid, more preferably contains at least one selected from glycerin fatty acid ester and sorbitan higher fatty acid ester, and still more preferably contains an ester compound of a polyhydric alcohol and a monohydric fatty acid.

Examples of the organic materials other than the organic polymer include waxes, oils, fatty acid metal salts, and amino acid compounds, and specific examples thereof include waxes such as carnauba wax, candelilla wax, beeswax, and higher alcohols; oils such as almond oil, olive oil, rice bran oil, squalane, mineral oil, alkane, and alkyl benzoate; fatty acids such as lauric acid, myristate, palmitic acid, stearic acid, 12-hydroxystearic acid, behenic acid, montanoic acid, and cerotic acid; fatty acid metal salts such as calcium laurate, zinc laurate, zinc myristate, zinc palmitate, magnesium stearate, zinc stearate, calcium stearate, aluminum stearate, calcium 12-hydroxystearic acid, zinc 12-hydroxystearic acid, magnesium 12-hydroxystearic acid, aluminum 12-hydroxystearic acid, calcium behenate, zinc behenate, magnesium behenate, calcium montanate, zinc montanate, magnesium montanate, and aluminum montanate; amino acid compounds such as N-lauroyl-L-arginine, N-lauroyl-L-lysine, N-hexanoyl-L-lysine, N-oleyl-L-lysine, N-palmitoyl-L-lysine, N-stearoyl-L-lysine, N-hexanoyl-L-lysine, N-myristonoyl-L-lysine, N-capryloyl-L-lysine, and N-decanoyl-L-lysine.

Examples of the inorganic materials include wollastonite, sericite, kaolin, mica, clay, talc, bentonite, smectite, alumina silicate, pyrophyllite, montmorillonite, calcium silicate, calcium carbonate, magnesium carbonate, dolomite, calcium sulfate, boron nitride, silicon carbide, magnesium silicate, calcium silicate, magnesium aluminometasilicate, magnesium aluminosilicate, hydroxyapatite, titanium oxide, silica, alumina, mica, titanium dioxide, zinc oxide, magnesium oxide, zinc oxide, hydrosaltite, and boron nitride.

The particles according to the present invention are not particularly limited, but it is preferable that the particles have the biodegradability since an environmental impact is reduced. The biodegradability of the particles can be achieved by including a compound having biodegradability as a component constituting the particles. Examples of the compound having the biodegradability include a surfactant, a cellulose, a cellulose resin, a polyester resin, a naturally derived wax, a naturally derived oil, polysaccharides, polyvinyl alcohol, polyalkylene oxide, and a biodegradable polymer among the constituent components of the particles.

The particles according to the present invention are not particularly limited, but it is preferable that a biodegradation rate after 10 days be 1% or more as measured in accordance with JIS K6950:2000. A lower limit of the biodegradation rate is preferably in the order of (1) 3%, (2) 5%, (3) 10%, (4) 15%, (5) 20%, (6) 25%, and (7) 30% (the larger the number in the parentheses, the better).

In the case where the particles according to the present invention contain the compound having the biodegradability, a weight ratio of the compound having the biodegradability to the particles is not particularly limited, but is preferably 30% by weight to 100% by weight. An upper limit of the weight ratio is more preferably 99.9% by weight, still more preferably 99.5% by weight, and particularly preferably 99.0% by weight. On the other hand, a lower limit of the weight ratio is preferably in the order of (1) 40% by weight, (2) 50% by weight, (3) 60% by weight, (4) 65% by weight, (5) 70% by weight, (6) 75% by weight, and (7) 80% by weight (the larger the number in the parentheses, the better). Furthermore, for example, 40% by weight to 100% by weight is more preferable, and 50% by weight to 100% by weight is still more preferable.

An oil absorption amount of the particles according to the present invention is not particularly limited, but is preferably 10 ml/100 g to 300 ml/100 g in terms of an excellent smooth texture when used in cosmetics. A lower limit of the oil absorption amount is preferably in the order of (1) 15 ml/100 g, (2) 20 ml/100 g, (3) 25 ml/100 g, (4) 30 ml/100 g, (5) 35 ml/100 g, and (6) 40 ml/100 g (the larger the number in the parentheses, the better). On the other hand, an upper limit of the oil absorption amount is preferably in the order of (1) 250 ml/100 g, (2) 200 ml/100 g, (3) 150 ml/100 g, (4) 120 ml/100 g, (5) 100 ml/100 g, (6) 90 ml/100 g, and (7) 85 ml/100 g (the larger the number in the parentheses, the better). Furthermore, for example, 20 ml/100 g to 300 ml/100 g is more preferable, and 30 ml/100 g to 200 ml/100 g is still more preferable.

The oil absorption amount of the particles is determined by the method described in Examples.

A water absorption amount of the particles according to the present invention is not particularly limited, but is preferably 10 ml/100 g to 300 ml/100 g in terms of an excellent dispersibility when used in an aqueous coating agent. A lower limit of the water absorption amount is preferably in the order of (1) 15 ml/100 g, (2) 20 ml/100 g, (3) 25 ml/100 g, (4) 30 ml/100 g, (5) 35 ml/100 g, and (6) 40 ml/100 g (the larger the number in the parentheses, the better). On the other hand, a preferable upper limit of the water absorption amount is preferably in the order of (1) 250 ml/100 g, (2) 200 ml/100 g, (3) 150 ml/100 g, (4) 120 ml/100 g, (5) 100 ml/100 g, (6) 90 ml/100 g, and (7) 85 ml/100 g (the larger the number in the parentheses, the better). Furthermore, for example, 20 ml/100 g to 300 ml/100 g is more preferable, and 30 ml/100 g to 200 ml/100 g is still more preferable.

The water absorption amount of the particles is determined by the method described in Examples.

(Method for Manufacturing Particles)

The particles according to the present invention can be manufactured, for example, by a method including a step 1 of mixing the components constituting the particles, the surfactant, the water-soluble polymer, and water to obtain a preliminary liquid mixture, a step 2 of heating and stirring the preliminary liquid mixture obtained in the step 1 to obtain a heated dispersion liquid, and a step 3 of cooling the heated dispersion liquid obtained in the step 2.

In the method for manufacturing the particles according to the present invention, in the case where an organic solvent is not used, particles having a low compressive aggregate modulus can be suitably manufactured, which is preferable. It is considered that by preparing the particles in water without using the organic solvent, the surfactant or the water-soluble polymer are likely to be present at an interface between the particles and the water at the time of particle formation, which contributes to improvement of surface properties of the particles. Furthermore, it is considered that when the particles are formed without using the organic solvent, polarity of a particle surface is appropriately maintained and the compressive recovery modulus is high. Further, In the case where the particles include the thermoplastic resin, it is considered that a lipophilic group of the surfactant affects a resin structure and further provides the soft texture. Furthermore, not using the organic solvent is environmentally friendly and preferred.

The components constituting the particles can be those described above.

As the surfactant, those described above can be used.

For the particles according to the present invention, it is preferable to mix the surfactant during the manufacture in order to manufacture particles having a low compressive aggregate modulus. The surfactant is not particularly limited, but from the viewpoint of achieving an effect of the present application, in the case where a nonionic surfactant and/or an anionic surfactant are used, the compressive aggregate modulus is low, which is preferable. In particular, in the case where a nonionic surfactant having an HLB value of 1 to 13 is used, the compressive aggregate modulus is low, which is preferable. The HLB value is more preferably 1 to 11, still more preferably 1 to 10, and particularly preferably 1.5 to 10. Further, it is preferable to use an ester surfactant or an ester salt surfactant since a soft feeling is obtained.

The HLB value can be calculated, for example, by the following Equation (3) using Griffin method.

$$\text{HLB} = 20 \times (\text{molecular weight of hydrophilic group/molecular weight of total}) \quad (3)$$

The surfactant may be finally contained in the particles. It is considered that in the case where the surfactant is present in the vicinity of the particle surface, the compressive aggregate modulus becomes lower. A weight ratio of the surfactant in the particles is not particularly limited, but is preferably 0.001 % by weight to 10% by weight in the terms of further reducing the compressive aggregate modulus, an upper limit of the weight ratio is more preferably 7% by weight, and still more preferably 5% by weight. On the other hand, a lower limit of the weight ratio is more preferably 0.005% by weight, and still more preferably 0.01% by weight. Furthermore, for example, 0.001% by weight to 7% by weight is more preferable, and 0.001% by weight to 5% by weight is still more preferable.

As the water-soluble polymer, those described above can be used.

For the particles according to the present invention, it is preferable to mix the water-soluble polymer during the manufacture in order to manufacture the particles having the low compressive aggregate modulus.

The water-soluble polymer may be finally contained in the particles. It is considered that in the case where the water-soluble polymer is present in the particles, the compressive aggregate modulus becomes lower. A weight ratio of the water-soluble polymer in the particles is not particularly limited, but is preferably 0.001% by weight to 10% by weight in the terms of further reducing the compressive aggregate modulus, an upper limit of the weight ratio is more preferably 8% by weight, and still more preferably 5% by weight. On the other hand, a lower limit of the weight ratio is more preferably 0.002% by weight, and still more preferably 0.005% by weight. Furthermore, for example, 0.001% by weight to 8% by weight is more preferable, and 0.001% by weight to 5% by weight is still more preferable.

The water-soluble polymer is not particularly limited, but is preferably selected depending on a particle size of the target particles. From the viewpoint of handling, it is preferable to use a water-soluble polymer having a viscosity of 2 mPa·s to 200,000 mPa·s at 20°C in a 4% aqueous solution.

For the particles according to the present invention, it is considered that in the case where the surfactant and the water-soluble polymer are mixed during the manufacture, the water-soluble polymer improves liquid viscosity during the manufacture, and the surfactant improves homogenization efficiency of the particles, and thus particles having a reduced surface roughness and a low compressive aggregate modulus are manufactured, which is further preferable. It is more preferable that the weight ratios of the surfactant and the water-soluble polymer are within the above-mentioned ranges, respectively, in the terms of further reducing the compressive aggregate modulus.

(Step 1)

The step 1 is a step of mixing a main component constituting the particles, the surfactant, the water-soluble polymer, and water to obtain the preliminary liquid mixture.

In the step 1, a total mixing ratio of the main component constituting the particles and the surfactant to water is not particularly limited, but it is preferable to mix the main component and the surfactant at a ratio of 1 part by weight to 200 parts by weight to 100 parts by weight of water, since uniformly shaped particles can easily be obtained. A lower limit of the mixing ratio is more preferably 3 parts by weight, still more preferably 5 parts by weight, and most preferably 10 parts by weight. On the other hand, an upper limit of the mixing ratio is more preferably 180 parts by weight, still more preferably 160 parts by weight, and most preferably 150 parts by weight. Further, for example, 5 parts by weight to 200 parts by weight is more preferable, and 10 parts by weight to 180 parts by weight is still more preferable.

In the step 1, a mixing ratio of the surfactant is not particularly limited, but it is preferable to mix the surfactant at a ratio of 0.001 parts by weight to 10 parts by weight to 100 parts by weight of the main component constituting the particles since the compressive aggregate modulus of the resulting particles is low. A lower limit of the mixing ratio is preferably 0.01 parts by weight, more preferably 0.05 parts by weight, and most preferably 0.1 parts by weight. An upper limit of the mixing ratio is preferably 7 parts by weight, more preferably 5 parts by weight, and most preferably 3 parts by weight. Further, for example, 0.001 parts by weight to 7 parts by weight is more preferable, and 0.01 parts by weight to 7 parts by weight is still more preferable.

In the step 1, a mixing ratio of the water-soluble polymer to water is not particularly limited, but it is preferable to mix the water-soluble polymer at a ratio of 0.1 parts by weight to 100 parts by weight to 100 parts by weight of water in the terms of the excellent dispersibility of the particles. A lower limit of the mixing ratio is more preferably 0.5 parts by weight, still more preferably 1 part by weight, and most preferably 2 parts by weight. On the other hand, an upper limit of the mixing ratio is more preferably 80 parts by weight, still more preferably 70 parts by weight, and most preferably 60 parts by weight. Further, for example, 0.5 parts by weight to 100 parts by weight is more preferable, and 1 part by weight to 100 parts by weight is still more preferable.

(Step 2)

The step 2 is a step of heating and stirring the preliminary liquid mixture obtained in the step 1 to obtain the heated dispersion liquid.

A pressure during the heating and the stirring is not particularly limited, but it is preferably carried out under pressure in the terms of easily obtaining uniformly distributed particles, and more preferably 0.1 MPa to 10 MPa. A lower limit of the pressure is more preferably a pressure equal to or higher than a saturated vapor pressure of water at a temperature during the heating.

A heating temperature is not particularly limited, but the temperature of 80°C to 300°C is preferable in the terms of a uniform particle shape. In the case where the thermoplastic resin is included as the main component constituting the particles, the temperature is preferably equal to or higher than a melting point of the thermoplastic resin. A more preferable temperature at the time of the heating and the stirring is a temperature higher than the melting point of the thermoplastic resin by 5°C or more, more preferably a temperature higher than the melting point of the thermoplastic resin by 10°C or more, and most preferably a temperature higher than the melting point of the thermoplastic resin by 15°C or more.

A stirring method is not particularly limited, as long as the mixture is stirred to the extent that the mixture is mixed.

A heating time is not particularly limited, but is preferably 1 hour to 30 hours in terms of the uniform particle shape. A lower limit of the heating time is more preferably 2 hours, still more preferably 3 hours, and most preferably 5 hours. An upper limit of the heating time is more preferably 25 hours, still more preferably 20 hours, and most preferably 15 hours.

(Step 3)

The step 3 is a step of cooling the heated dispersion liquid obtained in the step 2. By cooling the heated dispersion liquid in the step 2, a dispersion liquid of the particles can be obtained.

A cooling method is not particularly limited, but it is preferable to cool the heated dispersion liquid in the step 2 to 5°C to 50°C. A cooling rate is not particularly limited, and a rapid cooling may be performed using a cooling unit, or a natural cooling may be performed using air cooling. Although stirring conditions are not particularly limited, the stirring may be performed at a stirring speed in the step 2, or the stirring may be stopped.

The dispersion liquid after the cooling is an aqueous dispersion liquid containing the particles according to the present invention.

A use form of the particles according to the present invention may be a dispersion liquid, a wet powder, or a dry powder.

The wet powder can be obtained by, for example, dehydrating the dispersion liquid in the step 3 using a centrifugal separator, a pressurized press machine, or a vacuum hydro-extractor. It is easy to perform the dehydration treatment on the dispersion liquid in the step 3 after taking measures to lower the liquid viscosity. A method for lowering the liquid viscosity is not particularly limited, and examples thereof include a method for diluting the dispersion liquid by adding water, a method for salting out water-soluble components, and a method for decomposing water-soluble components with an oxidizing agent or an enzyme.

The dry powder can be obtained by drying the wet powder using a tray dryer, an indirect heating dryer, a fluidized bed dryer, a vacuum dryer, a vibration dryer, a pneumatic conveying dryer, or the like. Further, the dispersion liquid in the step 3 may be dried in a spray dryer, a fluidized bed dryer, or the like to obtain the dry powder.

The dry powder may be classified by air flow classification, screen classification, or the like.

The particles according to the present invention can be used for cosmetics, paints, optical applications, resins, building materials, and the like. Among them, the particles according to the present invention have an excellent slipperiness, and thus the particles can be suitably used in the cosmetics and the coating compositions. Further, since the particles have the soft feeling, a comfortable texture can be provided in the case where the particles are blended in the cosmetics.

In the case where the particles are used in the cosmetics, the particles can be used in combination with known cosmetic components. Examples of the cosmetic components include an oil agent, a surfactant, alcohols, water, a moisturizing agent, a gelling agent, a thickener, a powder other than the particles according to the present invention, an ultraviolet absorber, a preservative, an antibacterial agent, an antioxidant, and a functional component. Examples of a form of the cosmetic containing the particles according to the present invention include powder, solid, cream, gel, liquid, mousse, and spray. A content of the particles according to the present invention in the entire cosmetic is not particularly limited, but is preferably 0.1% by weight to 50% by weight, more preferably 0.5% by weight to 30% by weight, and still more preferably 1% by weight to 20% by weight.

In the case where the particles are used in the coating composition, the particles can be used in combination with known coating components. A content of the particles according to the present invention in the entire coating composition is not particularly limited, but is preferably 0.1% by weight to 30% by weight, more preferably 0.5% by weight to 20% by weight, and still more preferably 1% by weight to 10% by weight.

EXAMPLES

Examples of the particles according to the present invention will be specifically described below. The present invention is not limited to these Examples. Further, physical properties of the particles in Examples and Comparative Examples were measured and further evaluated in the following manner.

(Measurement of Particle Diameter)

Measurement was performed using a laser diffraction particle size distribution measuring apparatus (Microtrac particle size distribution meter (model 9320-HRA), manufactured by Nikkiso Co., Ltd.) by irradiating ultrasonic waves for 120 seconds using a wet measurement method. The average particle diameter (D50) was a value at a cumulative frequency of 50% based on the volume-based measurement, D90 was a value at a cumulative frequency of 90% based on the volume-based measurement, and D10 was a value at a cumulative frequency of 10% based on the volume-based measurement.

(Measurement of Compressive Aggregate Modulus and Compressive Recovery Modulus)

A sample was prepared by placing 30 mg of particles in an aluminum cup having a diameter of 6 mm (inner diameter of 5.65 mm) and a depth of 4.8 mm, and placing an aluminum lid having a diameter of 5.6 mm and a thickness of 0.1 mm on a top of a particle layer. Next, a force of 0.01 N was applied from the top of the aluminum lid using a pressurizer in an environment of 25°C using DMA (DMAQ800 type, manufactured by TAinstruments), and after standing for 5 minutes, an operation of pressurizing the particle layer from 0.01 N to 18 N at a rate of 10 N/min and an operation of depressurizing the particle layer from 18 N to 0.01 N at a rate of 10 N/min were repeated three times.

An aggregate modulus of the powder layer due to compression was calculated using the following Formula (4) from a height L1 ($\mu$m) of the particle layer after applying the force of 0.01 N and standing for 5 minutes, and a height L2 ($\mu$m) of the particle layer to which a force of 0.01 N was applied after repeating the pressurization and the depressurization three times. The closer the compressive aggregate modulus is to 0, the less the particle layer changes due to the compression, and the powder is less likely to aggregate.

The compressive recovery modulus of the particles was calculated from the following Equation (5) based on a strain S1 (%) of the particle layer in a state in which the force of 0.01 N for the third time was applied, a strain S2 (%) of the particle layer in a state in which the force of 18 N for the third time was applied after repeating the pressurization and the depressurization twice, and a strain S3 (%) of the particle layer in a state in which the force of 0.01 N was applied after repeating the pressurization and the depressurization three times. The closer the compressive recovery modulus is to 100%, the more cushioning property the particle layer has and the softer the texture is.

$$\text{Compressive aggregate modulus } (\%) = (1 - (\text{L2/L1})) \times 100 \quad (4)$$

$$\text{Compressive recovery modulus } (\%) = ((\text{S2} - \text{S3})/(\text{S2} - \text{S1})) \times 100 \quad (5)$$

(Measurement of Sphericity)

The particles were observed with a scanning electron microscope at a magnification of 1000 times, and a minor axis and a major axis of 30 any particles were measured. The minor axis/major axis was calculated for each particle, and an average value of 30 particles was defined as sphericity. For example, in the case where a ratio of the minor axis to the major axis is 1, the sphericity is 1.

(Measurement of Water Absorption Amount and Oil Absorption Amount)

Based on a method for measuring an oil absorption amount described in JIS-K5101, the water absorption amount was measured using deionized water instead of oil, and the oil absorption amount was measured using oleic acid.

(Evaluation of Coating Property)

When 0.05 g of particles were weighed out on an edge of a 10 cm x 5 cm piece of black artificial leather (trade name: SUPPLALE, manufactured by IDEATEX JAPAN Co.,Ltd.) and spread in one direction with a finger, the slipperiness, ease of spreading (uniformity), and the soft feeling were evaluated using the following criteria.

<Slipperiness>

A: The particles can be applied to the edge regardless of the amount of force.
B: The particles can be applied to the edge, but a difference is felt depending on the amount of force.
C: It is difficult to apply the particles to the edge.
D: The particles cannot be applied.

<Ease of Spreading>

A: The particles can be applied uniformly to the edge and have a uniform color.
B: The particles can be applied to the edge, but the color is slightly uneven.
C: It is difficult to uniformly apply the particles to the edge, resulting in uneven color.
D: The particles cannot be applied.

<Soft Feeling>

A: Softness is felt when the particles are applied.
B: Softness is slightly felt when the particles are applied.
C: Hardness is slightly felt when the particles are applied.
D: Hardness is felt when the particles are applied.

[Example 1]

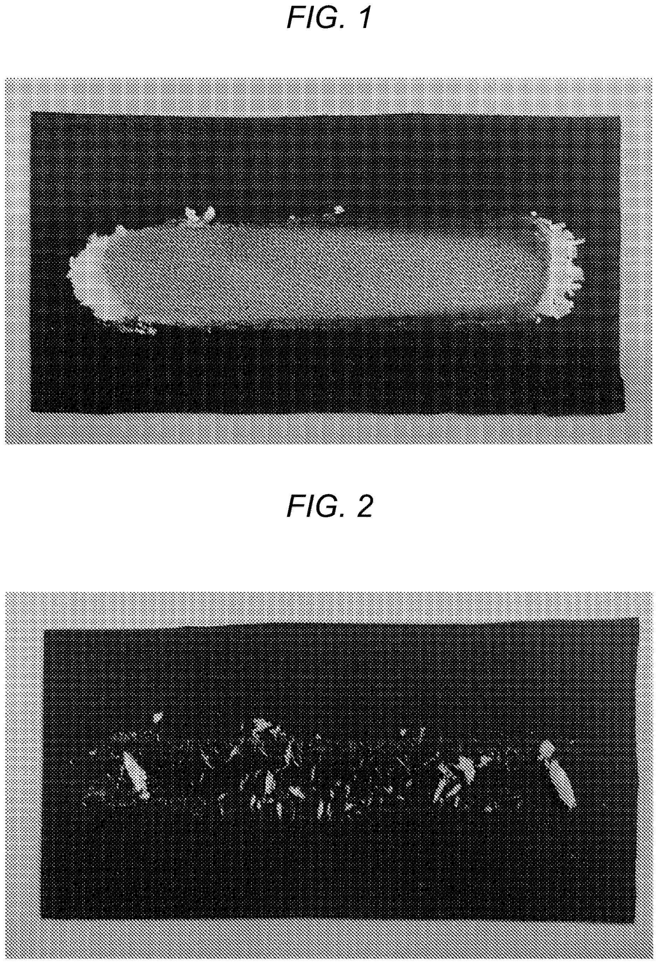

Three hundred parts by weight of water, 100 parts by weight of polybutylene succinate, 1 part by weight of sorbitan monolaurate (HLB value: 8.6), and 20 parts by weight of polyvinyl alcohol were mixed, and a mixture was charged into a 1 L pressure resistant vessel and sealed. An internal temperature of the vessel was raised to 140°C, the mixture was stirred at 400 rpm per minute under a pressure of 0.5 MPa for 3 hours, and then cooled to 50°C to obtain an aqueous dispersion liquid of particles.
An oxidizing agent was added to the aqueous dispersion liquid, the aqueous dispersion liquid was dehydrated by filtration, dried at 50°C, and classified to obtain particles 1. A content of the surfactant contained in the particles was 0.5%, and a content of polyvinyl alcohol was 0.3%. Physical properties of the obtained particles 1 are shown in Table 1. A photograph of the artificial leather after the coating property is evaluated is shown in FIG. 1.

[Example 2]

Three hundred parts by weight of water, 100 parts by weight of polybutylene succinate, 2 parts by weight of sorbitan monostearate (HLB value: 4.7), and 20 parts by weight of polyvinyl alcohol were mixed, and a mixture was charged into a 1 L pressure resistant vessel and sealed. An internal temperature of the vessel was raised to 160°C, the mixture was stirred at 400 rpm per minute under a pressure of 2.0 MPa for 5 hours, and then cooled to 50°C to obtain an aqueous dispersion liquid of particles.
An oxidizing agent was added to the aqueous dispersion liquid, the aqueous dispersion liquid was dehydrated by filtration, dried at 50°C, and classified to obtain particles 2. A content of the surfactant contained in the particles was 0.9%, and a content of polyvinyl alcohol was 0.4%. Physical properties of the obtained particles 2 are shown in Table 1.

[Example 3]

Three hundred parts by weight of water, 100 parts by weight of polybutylene succinate adipate, 2 parts by weight of sorbitan monolaurate (HLB value: 8.6), and 20 parts by weight of polyvinyl alcohol were mixed, and a mixture was charged into a 1 L pressure resistant vessel and sealed. An internal temperature of the vessel was raised to 140°C, the mixture was stirred at 400 rpm per minute under a pressure of 0.5 MPa for 3 hours, and then cooled to 50°C to obtain an aqueous dispersion liquid of particles.
An oxidizing agent was added to the aqueous dispersion liquid, the aqueous dispersion liquid was dehydrated by filtration, dried at 50°C, and classified to obtain particles 3. A content of the surfactant contained in the particles was 1.0%, and a content of polyvinyl alcohol was 0.2%. Physical properties of the obtained particles 3 are shown in Table 1.

[Example 4]

Three hundred parts by weight of water, 50 parts by weight of polybutylene succinate adipate, 50 parts by weight of polybutylene succinate, 0.1 parts by weight of sorbitan monostearate (HLB value: 4.7), 0.1 parts by weight of diethylhexyl sodium sulfosuccinate, and 20 parts by weight of polyvinyl alcohol were mixed, and a mixture was charged into a 1 L pressure resistant vessel and sealed. An internal temperature of the vessel was raised to 140°C, the mixture was stirred at 400 rpm per minute under a pressure of 0.5 MPa for 10 hours, and then cooled to 50°C to obtain an aqueous dispersion liquid of particles.
An oxidizing agent was added to the aqueous dispersion liquid, the aqueous dispersion liquid was dehydrated by filtration, dried at 50°C, and classified to obtain particles 4. A content of the surfactant contained in the particles was 0.001%, and a content of polyvinyl alcohol was 0.05%. Physical properties of the obtained particles 4 are shown in Table 1.

[Example 5]

Three hundred parts by weight of water, 80 parts by weight of polyhydroxyalkanoate, 20 parts by weight of polybutylene succinate, 3 parts by weight of sorbitan monolaurate (HLB value: 8.6), and 20 parts by weight of polyvinyl alcohol were mixed, and a mixture was charged into a 1 L pressure resistant vessel and sealed. An internal temperature of the vessel was raised to 160°C, the mixture was stirred at 400 rpm per minute under a pressure of 1.0 MPa for 10 hours, and then cooled to 50°C to obtain an aqueous dispersion liquid of particles.

An oxidizing agent was added to the aqueous dispersion liquid, the aqueous dispersion liquid was washed with a large amount of water, dehydrated by filtration, dried at 50°C, and classified to obtain particles 5. A content of the surfactant contained in the particles was 0.8%, and a content of polyvinyl alcohol was 0%. Physical properties of the obtained particles 5 are shown in Table 1.

[Example 6]

Three hundred parts by weight of water, 100 parts by weight of polybutylene succinate adipate, 1 part by weight of sorbitan monostearate (HLB value: 4.7), 1 part by weight of fumed silica, and 20 parts by weight of polyvinyl alcohol were mixed, and a mixture was charged into a 1 L pressure resistant vessel and sealed. An internal temperature of the vessel was raised to 120°C, the mixture was stirred at 400 rpm per minute under a pressure of 0.5 MPa for 3 hours, and then cooled to 50°C to obtain an aqueous dispersion liquid of particles.
An oxidizing agent was added to the aqueous dispersion liquid, the aqueous dispersion liquid was dehydrated by filtration, dried at 50°C, and classified to obtain particles 6. A content of the surfactant contained in the particles was 0.7%, and a content of polyvinyl alcohol was 2.0%. Physical properties of the obtained particles 6 are shown in Table 1.

[Example 7]

Three hundred parts by weight of water, 50 parts by weight of polybutylene adipate terephthalate, 50 parts by weight of polybutylene succinate, 0.5 parts by weight of sorbitan monolaurate (HLB value: 8.6), and 20 parts by weight of polyvinyl alcohol were mixed, and a mixture was charged into a 1 L pressure resistant vessel and sealed. An internal temperature of the vessel was raised to 200°C, the mixture was stirred at 400 rpm per minute under a pressure of 2.0 MPa for 5 hours, and then cooled to 50°C to obtain an aqueous dispersion liquid of particles.
An oxidizing agent was added to the aqueous dispersion liquid, the aqueous dispersion liquid was dehydrated by filtration, dried at 50°C, and classified to obtain particles 7. A content of the surfactant contained in the particles was 0.01%, and a content of polyvinyl alcohol was 0.5%. Physical properties of the obtained particles 7 are shown in Table 1.

[Example 8]

Three hundred parts by weight of water, 30 parts by weight of cellulose powder (average particle diameter 7.5 $\mu$m), 70 parts by weight of polybutylene succinate, 1 part by weight of sorbitan monolaurate (HLB value: 8.6), and 20 parts by weight of polyvinyl alcohol were mixed, and a mixture was charged into a 1 L pressure resistant vessel and sealed. An internal temperature of the vessel was raised to 140°C, the mixture was stirred at 400 rpm per minute under a pressure of 1.0 MPa for 10 hours, and then cooled to 50°C to obtain an aqueous dispersion liquid of particles.
An oxidizing agent was added to the aqueous dispersion liquid, the aqueous dispersion liquid was washed with a large amount of water, dehydrated by filtration, dried at 50°C, and classified to obtain particles 8. A content of the surfactant contained in the particles was 0%, and a content of polyvinyl alcohol was 0%. Physical properties of the obtained particles 8 are shown in Table 1.

[Example 9]

Three hundred parts by weight of water, 100 parts by weight of polybutylene adipate terephthalate, 0.3 parts by weight of glyceryl monooleate (HLB value: 2.8), and 30 parts by weight of polyvinyl alcohol were mixed, and a mixture was charged into a 1 L pressure resistant vessel and sealed. An internal temperature of the vessel was raised to 140°C, the mixture was stirred at 400 rpm per minute under a pressure of 1.0 MPa for 10 hours, and then cooled to 50°C to obtain an aqueous dispersion liquid of particles.
An oxidizing agent was added to the aqueous dispersion liquid, the aqueous dispersion liquid was washed with a large amount of water, dehydrated by filtration, dried at 50°C, and classified to obtain particles 9. A content of the surfactant contained in the particles was 0.005%, and a content of polyvinyl alcohol was 0.001%. Physical properties of the obtained particles 9 are shown in Table 1.

[Example 10]

Three hundred parts by weight of water, 70 parts by weight of polyethylene, 30 parts by weight of ethylene-methyl methacrylate copolymer, 0.1 parts by weight of sorbitan monostearate (HLB value: 4.7), 0.3 parts by weight of diethylhexyl sodium sulfosuccinate, and 40 parts by weight of polyvinyl alcohol were mixed, and a mixture was charged into a 1 L pressure resistant vessel and sealed. An internal temperature of the vessel was raised to 150°C, the mixture was stirred at 400 rpm per minute under a pressure of 1.0 MPa for 3 hours, and then cooled to 50°C to obtain an aqueous dispersion liquid of particles.

An oxidizing agent was added to the aqueous dispersion liquid, the aqueous dispersion liquid was washed with a large amount of water, dehydrated by filtration, dried at 50°C, and classified to obtain particles 10. A content of the surfactant contained in the particles was 0%, and a content of polyvinyl alcohol was 0.01%. Physical properties of the obtained particles 10 are shown in Table 1.

[Comparative Example 1]

Polylactic acid particles (trade name: ECOBEADS D-5, manufactured by DAITO KASEI KOGYO CO., LTD.) were measured and evaluated in the same manner. Physical properties are shown in Table 2. A photograph of the artificial leather after the coating property is evaluated is shown in FIG. 2.

[Comparative Example 2]

Cellulose particles (trade name: CELLULOBEADS D-5, manufactured by DAITO KASEI KOGYO CO., LTD.) were measured and evaluated in the same manner. Physical properties are shown in Table 2.

[Comparative Example 3]

Ten parts by weight of polylactic acid, 16 parts by weight of hydroxypropyl-cellulose, and 300 parts by weight of tetrahydrofuran were mixed, and a mixture was charged into a 1 L flask, heated to 60°C, and stirred until a polymer was dissolved. A temperature was lowered to 25°C, and 330 parts by weight of water was added dropwise while stirring. After an entire amount of water was added, the mixture was stirred for 30 minutes, washed with a large amount of water, dehydrated by filtration, and dried under vacuum at 80°C to obtain particles 11. Physical properties of the obtained particles 11 are shown in Table 2.

[Comparative Example 4]

Twenty parts by weight of polybutylene succinate and 600 parts by weight of 3-methoxy-3-methyl-1-butanol were mixed, and a mixture was charged into a 1 L pressure resistant vessel and sealed. An internal temperature of the vessel was raised to 120°C, the mixture was stirred at 600 rpm per minute under a pressure of 0.5 MPa for 1 hour, and then cooled to 25°C to obtain an aqueous dispersion liquid of particles. The aqueous dispersion liquid was deliquified by filtration and dried at 50°C to obtain particles 12. Evaluation results of the obtained particles 12 are shown in Table 2.

Table 1

| | | Example | | | | | | | | | |
|---|---|---|---|---|---|---|---|---|---|---|---|
| | | 1 | 2 | 3 | 4 | 5 | 6 | 7 | 8 | 9 | 10 |
| Average particle diameter (D50) (μm) | | 10.8 | 7.5 | 4.2 | 19.7 | 3.8 | 13.9 | 28.2 | 10.1 | 43.7 | 8.5 |
| D90/D50 | | 1.74 | 2.06 | 1.56 | 2.13 | 1.55 | 1.96 | 1.92 | 2.56 | 2.73 | 2.68 |
| D10/D50 | | 0.44 | 0.49 | 0.40 | 0.54 | 0.59 | 0.48 | 0.51 | 0.41 | 0.42 | 0.38 |
| Compressive aggregate modulus (%) | | 4.8 | 9.8 | 11.5 | 14.0 | 9.6 | 9.3 | 5.3 | 20.5 | 13.2 | 10.3 |
| Compressive recovery modulus (%) | | 90.9 | 88.6 | 88.7 | 91.3 | 84.4 | 93.9 | 85.6 | 72.3 | 80.5 | 78.8 |
| Sphericity | | 0.95 | 0.95 | 0.96 | 0.92 | 0.92 | 0.78 | 0.87 | 0.68 | 0.89 | 0.88 |
| Water absorption amount (ml/100 g) | | 50 | 60 | 78 | 52 | 82 | 55 | 45 | 103 | 55 | 54 |
| Oil absorption amount (ml/100 g) | | 55 | 65 | 88 | 53 | 93 | 60 | 48 | 72 | 60 | 61 |
| Coating property | Slipperiness | A | A | B | B | B | A | A | B | B | B |
| | Soft feeling | A | A | A | A | A | A | B | B | B | B |
| | Spreading property | A | A | A | A | A | A | A | B | B | B |

Table 2

| | | Comparative Example | | | |
|---|---|---|---|---|---|
| | | 1 | 2 | 3 | 4 |
| Average particle diameter (D50) ($\mu$m) | | 7.2 | 9.3 | 12.5 | 35.6 |
| D90/D50 | | 2.27 | 1.90 | 1.29 | 3.57 |
| D10/D50 | | 0.47 | 0.44 | 0.80 | 0.25 |
| Compressive aggregate modulus (%) | | 26.3 | 8.2 | 25.4 | 26.5 |
| Compressive recovery modulus (%) | | 38.8 | 53.8 | 44.6 | 55.6 |
| Sphericity | | 0.90 | 0.72 | 0.84 | 0.70 |
| Water absorption amount (ml/100 g) | | 60 | 173 | 88 | 195 |
| Oil absorption amount (ml/100 g) | | 55 | 50 | 92 | 210 |
| Coating property | Slipperiness | D | C | C | D |
| | Soft feeling | D | C | D | C |
| | Spreading property | D | C | C | D |

The particles in Examples 1 to 10 are particles of the invention of the first aspect of the present application and/or particles of the invention of the second aspect thereof, and thus the particles had an excellent slipperiness and coating property, and had a soft texture. Further, it was found that an excellent slipperiness property, an excellent coating property and a soft texture was provided on the artificial leather which was a soft material and had a texture similar to a human skin. In FIG. 1, it was also visually found that the particles were uniformly coated on the artificial leather.

On the other hand, in the particles of Comparative Examples 1, 3, and 4, the compressive aggregate modulus was not in a range of 0% to 25%, and the compressive recovery modulus was not in a range of 60% to 100%, and thus the slipperiness property and the coating property were poor. Further, in a coating property evaluation of the particles of Comparative Example 1, the powder layer only moved in lumps, and the particles could not be applied uniformly on the artificial leather. Furthermore, the particles in Comparative Example 2 were particles having a compressive aggregate modulus within the range of 0% to 25% but not including a thermoplastic resin, and the compressive recovery modulus was not within the range of 60% to 100%, and thus the particles had a hard powdery feel, and a poor slipperiness property and a poor coating property.

The particles according to the present invention exhibit specific physical properties, are excellent in slipperiness property and coating property, and have a soft texture.

INDUSTRIAL APPLICABILITY

The particles according to the present invention are useful as a compounding agent in cosmetics since the particles have an excellent slipperiness property and an excellent coating property on an artificial leather having a texture similar to a human skin, and have a soft texture. Further, since the particles have the excellent coating property even when the particles are applied to a soft material such as an artificial leather, the particles are useful as a compounding agent in a coating agent.

The particles according to the present invention have the excellent slipperiness property and excellent coating property, and have the soft texture, and thus the particles can be used as the compounding agent in various products such as cosmetics, paints, coating compositions, films, and molded bodies.

**Claims**

1.  Particles having an average particle diameter of 0.5 $\mu$m to 100 $\mu$m, a compressive aggregate modulus of 0% to 25%, and a compressive recovery modulus of 60% to 100%.

2.  The particles according to claim 1, comprising:
    an organic polymer.

3. The particles according to claim 2, wherein the organic polymer comprises a thermoplastic resin.

4. Particles having an average particle diameter of 0.5 μm to 100 μm and a compressive aggregate modulus of 0% to 25%, the particles comprising:
   a thermoplastic resin.

5. The particles according to claim 3 or 4, wherein the thermoplastic resin comprises at least one selected from a polyvinyl resin, a polyacrylic resin, a polystyrene resin, a polyolefin resin, a polyester resin, a polyether resin, a polyamide resin, a thermoplastic polyurethane resin, and a cellulose resin.

6. The particles according to any one of claims 1 to 5, having a sphericity of 0.6 to 1.0.

7. The particles according to any one of claims 1 to 6, having a value (D90/D50) of 1.0 to 3.5 where the value (D90/D50) is obtained by dividing a particle diameter (D90) at a cumulative frequency of 90% by volume-based measurement by the average particle diameter (D50).

8. The particles according to any one of claims 2 to 7, wherein the particles have biodegradability.

9. A cosmetic comprising:
   the particles according to any one of claims 1 to 8.

10. A coating composition comprising:
    the particles according to any one of claims 1 to 8.

## FIG. 1

## FIG. 2

# EP 4 442 736 A1

## INTERNATIONAL SEARCH REPORT

International application No.

**PCT/JP2022/046119**

### A. CLASSIFICATION OF SUBJECT MATTER

*C08J 3/12*(2006.01)i; *A61K 8/00*(2006.01)i; *A61K 8/73*(2006.01)i; *A61K 8/81*(2006.01)i; *A61K 8/85*(2006.01)i; *A61K 8/86*(2006.01)i; *A61K 8/87*(2006.01)i; *A61K 8/88*(2006.01)i; *C08J 3/16*(2006.01)i; *C09D 7/65*(2018.01)i; *C09D 201/00*(2006.01)i

FI: C08J3/12 Z CER; A61K8/00; A61K8/73; A61K8/81; A61K8/85; A61K8/86; A61K8/87; A61K8/88; C08J3/12 CEZ; C08J3/16 CFD; C09D7/65; C09D201/00

According to International Patent Classification (IPC) or to both national classification and IPC

### B. FIELDS SEARCHED

Minimum documentation searched (classification system followed by classification symbols)

C08J3/00 - 3/28, C08J99/00, A61K8/00 - 8/99, A61Q1/00 - 90/00, C09D1/00 - 10/00, C09D101/00 - 201/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 63-010603 A (MITSUBISHI RAYON CO LTD) 18 January 1988 (1988-01-18) claims, p. 2, upper right column, line 4 to lower left column, line 8, p. 2, lower left column, line 17 to lower right column, line 5, p. 4, upper left column, lines 11-19, examples | 1-7, 9-10 |
| A | | 8 |
| A | JP 04-317784 A (NIPPON SHOKUBAI CO LTD) 09 November 1992 (1992-11-09) entire text | 1-10 |
| A | JP 06-313008 A (MITSUBISHI PETROCHEM CO LTD) 08 November 1994 (1994-11-08) entire text | 1-10 |
| A | JP 04-335035 A (MITSUI PETROCHEM IND LTD) 24 November 1992 (1992-11-24) entire text | 1-10 |

☑ Further documents are listed in the continuation of Box C.   ☑ See patent family annex.

| | | |
|---|---|---|
| \* | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **22 February 2023** | **07 March 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

EP 4 442 736 A1

**INTERNATIONAL SEARCH REPORT**

| International application No. |
|---|
| **PCT/JP2022/046119** |

**C. DOCUMENTS CONSIDERED TO BE RELEVANT**

| Category* | Citation of document, with indication, where appropriate, of the relevant passages | Relevant to claim No. |
|---|---|---|
| A | JP 2003-321574 A (NIPPON ASAHI KIKO HANBAI KK) 14 November 2003 (2003-11-14)<br>entire text | 1-10 |
| A | JP 2006-299234 A (NIPPON SHOKUBAI CO LTD) 02 November 2006 (2006-11-02)<br>entire text | 1-10 |
| A | WO 2020/213298 A1 (SANYO CHEMICAL IND LTD) 22 October 2020 (2020-10-22)<br>entire text | 1-10 |
| A | JP 2007-326935 A (SEKISUI CHEMICAL CO LTD) 20 December 2007 (2007-12-20)<br>entire text | 1-10 |
| A | WO 2009/113678 A1 (NIPPON SHOKUBAI CO LTD) 17 September 2009 (2009-09-17)<br>entire text | 1-10 |
| A | JP 2018-095794 A (SEKISUI PLASTICS) 21 June 2018 (2018-06-21)<br>entire text | 1-10 |
| A | JP 2021-041650 A (TOPPAN PRINTING CO LTD) 18 March 2021 (2021-03-18)<br>entire text | 1-10 |
| A | US 2014/0005377 A1 (COVIDIEN LP, CONFLUENT SURGICAL, INC.) 02 January 2014<br>(2014-01-02)<br>whole document | 1-10 |

Form PCT/ISA/210 (second sheet) (January 2015)

## INTERNATIONAL SEARCH REPORT
### Information on patent family members

| | International application No. |
|---|---|
| | **PCT/JP2022/046119** |

| Patent document cited in search report | | | Publication date (day/month/year) | Patent family member(s) | | | Publication date (day/month/year) |
|---|---|---|---|---|---|---|---|
| JP | 63-010603 | A | 18 January 1988 | (Family: none) | | | |
| JP | 04-317784 | A | 09 November 1992 | (Family: none) | | | |
| JP | 06-313008 | A | 08 November 1994 | (Family: none) | | | |
| JP | 04-335035 | A | 24 November 1992 | (Family: none) | | | |
| JP | 2003-321574 | A | 14 November 2003 | (Family: none) | | | |
| JP | 2006-299234 | A | 02 November 2006 | US 2006/0183828 A1 whole document WO 2006/088115 A1 whole document EP 1690887 A1 whole document | | | |
| WO | 2020/213298 | A1 | 22 October 2020 | US 2022/0212165 A1 whole document EP 3957679 A1 whole document | | | |
| JP | 2007-326935 | A | 20 December 2007 | US 2009/0042307 A1 whole document WO 2007/063701 A1 whole document EP 1955764 A1 whole document | | | |
| WO | 2009/113678 | A1 | 17 September 2009 | US 2011/0166300 A1 whole document EP 2253375 A1 whole document | | | |
| JP | 2018-095794 | A | 21 June 2018 | (Family: none) | | | |
| JP | 2021-041650 | A | 18 March 2021 | (Family: none) | | | |
| US | 2014/0005377 | A1 | 02 January 2014 | EP 2679601 A1 whole document | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012105140 A

- WO 2017056908 A